# EUROPÄISCHE PATENTANMELDUNG

(11) **EP 3 318 286 A1**
(43) Veröffentlichungstag der Anmeldung: **09.05.2018**
(21) Anmeldenummer: 16197307.8
(22) Anmeldetag: 04.11.2016
(51) Int. Cl.: A61L 2/18, A47L 15/42, A47L 15/48

(54) **REINIGUNGS- UND DESINFEKTIONSGERÄT SOWIE VERFAHREN ZUM DESINFIZIEREN EINES VON EINEM FLUID DURCHSTRÖMBAREN BEREICHS EINES SOLCHEN GERÄTS**

(71) Anmelder: MELAG Medizintechnik oHG, 10829 Berlin (DE)
(72) Erfinder: LITZBA, Guido, 14513 Teltow (DE); HÖRNING, Andreas, 10969 Berlin (DE)
(74) Vertreter: Maikowski & Ninnemann Patentanwälte Partnerschaft mbB

(57) **Zusammenfassung**

Die Erfindung betrifft ein Reinigungs- und Desinfektionsgerät für medizinische und/oder zahnmedizinische Instrumente, mit einer Medientrenneinrichtung (5) und einer Aufbereitungskammer (10), wobei die Medientrenneinrichtung (5) einen ersten Einlass (4) für Spülflüssigkeit, einen zweiten Einlass (6) für Trocknungsluft sowie einen gemeinsamen Auslass (16) für Spülflüssigkeit und Trocknungsluft aufweist, durch den hindurch wahlweise Spülflüssigkeit oder Trocknungsluft aus der Medientrenneinrichtung (5) strömen kann. Das Reinigungs- und Desinfektionsgerät (1) zeichnet sich erfindungsgemäß dadurch aus, dass die Medientrenneinrichtung (5) eine Vorrichtung zur Erzeugung eines Unterdrucks aufweist, mittels der ein nicht nur flüssiges Fluid in die Medientrenneinrichtung gesaugt werden kann. Die Erfindung betrifft ferner ein Verfahren zum Desinfizieren eines von einem Fluid durchströmbaren Bereichs eines solchen Reinigungs- und Desinfektionsgeräts (1).

## Beschreibung

Die vorliegende Erfindung betrifft ein Reinigungs- und Desinfektionsgerät gemäß dem Oberbegriff des Anspruchs 1 sowie ein Verfahren zum Desinfizieren eines von einem Fluid durchströmbaren Bereichs eines Reinigungs- und Desinfektionsgeräts gemäß dem Oberbegriff des Anspruchs 14.

Reinigungs- und Desinfektionsgeräte mit aktiver Trocknung enthalten meist ein Heißluftgebläse zur Beschleunigung der Trocknung bzw. Reduzierung der Restfeuchtigkeit. Dabei wird grundsätzlich zwischen der Innen- und Außentrocknung der in dem Reinigungs-und Desinfektionsgerät aufbereiteten Instrumente unterschieden. Bei der Innentrocknung werden Instrumente, die ein Lumen aufweisen, innerhalb des Lumens mit Luft durchgeblasen. Die Trocknung wird durch die Eigenwärme und die einströmende Heißluft beschleunigt. Hierbei wird eine schnellstmögliche Trocknung angestrebt. Bei der Außentrocknung strömt Heißluft in die Aufbereitungskammer des Reinigungs- und Desinfektionsgeräts und unterstützt zusätzlich zur Eigenwärme der Instrumente die Trocknung der Außenflächen der Instrumente.

Aus der DE 25 56 035 A1 ist eine Spülmaschine mit einem Trocknungsluftgebläse bekannt, bei der ein Auslass des Trocknungsluftgebläses über ein Ventil abgesperrt werden kann. Eine Pumpe zum Umwälzen von Spülflüssigkeit kann nur dann aktiviert werden, wenn das Ventil am Auslass des Trocknungsluftgebläses geschlossen ist. Umgekehrt kann das Trocknungsluftgebläse nur dann aktiviert werden, wenn das Ventil geöffnet ist. Bei etwaigen Abnutzungserscheinungen des Ventils nach einer bestimmten Betriebsdauer können Undichtigkeiten am Ventil auftreten. Wenn diese nicht detektiert werden, kann es zu einem Kurzschluss der zum Erwärmen der Trocknungsluft eingesetzten Trocknungsheizung kommen. Ferner ist zu berücksichtigen, dass sich vor dem Ventil eine Sackstelle befindet, in der sich Spülflüssigkeit ansammeln kann. Wenn die Spülmaschine auch zu Desinfektionszwecken eingesetzt werden soll, kann eine ausreichende Desinfektionswirkung wegen einer entsprechenden Spülflüssigkeitsansammlung bei gleichzeitig fehlender Spülflüssigkeitsbewegung an der Sackstelle nicht mehr garantiert werden. Sich am Ventil ansammelnde Leckageflüssigkeit würde während des Trocknungsprozesses in die Aufbereitungskammer geblasen werden und dort zu einer Rekontamination des Aufbereitungsguts führen.

Die EP 2 641 619 A1 beschreibt einen Reinigungs- und Desinfektionsautomaten, bei dem eine Trenn- und Verteileinrichtung zur Beschickung einer Fluidabgabeeinrichtung wahlweise mit Spülflotte oder mit Trocknungsluft vorgesehen ist. Die Trenn- und Verteileinrichtung ist mit zwei Kammern ausgestattet, von denen eine der Zuführung von Spülflüssigkeit und die andere der Zuführung von Trocknungsluft dient. Dabei ist insbesondere ein zusätzlicher Ausgangsanschluss in der zweiten Kammer, die zur Zufuhr von Trocknungsluft vorgesehen ist, ausgebildet, um ein Durchspülen dieser zweiten Kammer mit Spülflüssigkeit zu ermöglichen. Dieser zusätzliche Ausgangsanschluss kann in Abhängigkeit von der Art der Beladung abgeschottet bzw. verdeckt werden, wobei in diesem Fall nicht ausreichend Spülflüssigkeit in die Trenn- und Verteileinrichtung gelangt, um die zweite Kammer durchzuspülen. Das heißt, eine für die Vermeidung einer Keimbildung in der zweiten Kammer erforderliche ausreichende Durchspülung kann in Abhängigkeit der gewählten Beladung nicht immer gewährleistet werden. Durch den teilweise verschließbaren weiteren Ausgangsanschluss werden zudem die Strömungsverhältnisse von Trocknungsluft, die für die Außentrocknung von in dem Reinigungs- und Desinfektionsautomaten aufbereiteten Instrumenten eingesetzt wird, beeinflusst. Dadurch können größere Mengen an Restfeuchtigkeit auf den Instrumenten zurückbleiben. Außerdem resultiert bei dieser Lösung eine schlechte Innentrocknung der Instrumente aufgrund des nur teilweise verdeckten zusätzlichen Ausgangsanschlusses. Denn durch die nur teilweise Abdeckung resultiert ein Druckabfall der zur Trocknung eingesetzten Luft. Schließlich ist es bei dieser Ausgestaltung möglich, dass in Abhängigkeit der Beladung sehr viel Spülflüssigkeit in die Trenn- und Verteileinrichtung eintritt, wodurch sich ein Druck an der in der zweiten Kammer angeordneten Luftklappe aufbaut. Aufgrund von betriebsbedingtem Verschleiß oder aufgrund von Verschmutzungen können so Leckagen an der Luftklappe entstehen. Sich ansammelnde Leckageflüssigkeit kann nicht desinfiziert werden und wird während der Trocknung auf die bereits desinfizierte Beladung geblasen, die dadurch rekontaminiert wird.

Aus der DE 10 2013 100 995 A1 ist ein Ventilgehäuse eines Rückschlagventils bekannt, das einteilig mit einem Verteiler ausgebildet ist. Ferner wird in dieser deutschen Patentanmeldung beschrieben, dass zwei solche Verteiler die Funktionalität einer Medientrenneinrichtung bereitstellen. Dabei schließt ein Rückschlagventil beim Umwälzen von Spülflüssigkeit den Trocknungsluftanschluss, während das andere Rückschlagventil beim Trocknen den Anschluss für die Spülflüssigkeit schließt. Hierbei handelt es sich allerdings um eine sehr platzraubende Lösung. Wenn sich zudem an dem Luftrückschlagventil Leckageflüssigkeit ansammelt, wird diese während der Trocknung auf die Beladung geblasen, was zu einer Rekontamination der Beladung führt. Darüber hinaus kann durch Leckagen ein Kurzschluss in der Trocknungsheizung entstehen.

Der vorliegenden Erfindung liegt die Aufgabe zugrunde, ein Reinigungs- und Desinfektionsgerät anzugeben, das einen Medientrenner aufweist, der die aus dem Stand der Technik bekannten Nachteile überwindet. Insbesondere soll eine rekontaminationsfreie Trocknungsluftzufuhr durch den Medientrenner auf im Reinigungs- und Desinfektionsgerät angeordnete Instrumente ermöglicht werden. Der Erfindung liegt ferner die Aufgabe zugrunde, ein Verfahren zur rekontaminationsfreien Trocknungsluftzufuhr in die Aufbereitungskammer eines Reinigungs- und Desinfektionsgeräts bereitzustellen.

Diese Aufgabe wird mit einem Reinigungs- und Desinfektionsgerät für medizinische und/oder zahnmedizinische Instrumente mit den Merkmalen des Anspruchs 1 gelöst. Ein solches Gerät weist eine Medientrenneinrichtung und eine Aufbereitungskammer auf. Die Medientrenneinrichtung weist einen ersten Einlass für Spülflüssigkeit auf und einen zweiten Einlass für Trocknungsluft auf. Ferner umfasst die Medientrenneinrichtung einen gemeinsamen Auslass für Spülflüssigkeit und Trocknungsluft. Durch diesen gemeinsamen Auslass kann entweder Spülflüssigkeit oder Trocknungsluft aus der Medientrenneinrichtung strömen.

Erfindungsgemäß zeichnet sich die Medientrenneinrichtung dadurch aus, dass sie zusätzlich eine Vorrichtung zur Erzeugung eines Unterdrucks aufweist. Mit dieser Vorrichtung kann ein Fluid, das keine reine Flüssigkeit ist, in die Medientrenneinrichtung, insbesondere in einen Trocknungsluftzufuhrbereich der Medientrenneinrichtung, gesaugt werden. Auf diese Weise lässt sich eine Desinfektion auch solcher Bereiche der Medientrenneinrichtung erreichen, die üblicherweise nicht von heißer Spülflüssigkeit durchströmt werden. In einer Variante lässt sich das Fluid nicht nur in die Medientrenneirichtung hinein, sondern durch die Medientrenneinrichtung hindurch saugen; das heißt, es kann auch wieder aus der Medientrenneinrichtung austreten. In einer Variante wird das nicht nur flüssige Fluid aus der Aufbereitungskammer des Reinigungs- und Desinfektionsgeräts gesaugt.

Bei dem durch die Vorrichtung zur Erzeugung eines Unterdrucks angesaugten Fluid handelt es sich insbesondere um ein gasförmiges Fluid oder eine Mischung aus einem gasförmigen Fluid und einem flüssigen Fluid. Beispielsweise kann Wasserdampf oder nebelartige feucht-heiße Luft aus der Aufbereitungskammer angesaugt werden. Das heißt, das angesaugte Fluid weist insbesondere desinfizierende Eigenschaften auf.

In einer Variante ist die Vorrichtung zur Erzeugung eines Unterdrucks im gemeinsamen Auslass der Medientrenneinrichtung ausgebildet. Dabei ist es insbesondere vorgesehen, dass auch ein von dem angesaugten Fluid durchströmter Strömungspfad strömungstechnisch mit dem gemeinsamen Auslass verbunden ist. Wenn dann Spülflüssigkeit durch die Medientrenneinrichtung strömt und die Medientrenneinrichtung durch den gemeinsamen Auslass verlässt, wird das angesaugte und durch den Strömungspfad strömende Fluid automatisch mit der Spülflüssigkeit im Bereich des gemeinsamen Auslasses vereinigt. Dann lässt sich das angesaugte Fluid besonders einfach wieder in die Aufbereitungskammer des Reinigungs- und Desinfektionsgeräts zurückführen.

In einer Variante ist die Vorrichtung zur Erzeugung eines Unterdrucks als Venturi-Düse ausgestaltet. Beispielsweise kann eine Verengung im gemeinsamen Auslass ausgebildet sein, sodass durch den gemeinsamen Auslass aus der Medientrenneinrichtung strömende Spülflüssigkeit durch die Verengung beschleunigt wird und damit selbst einen Unterdruck erzeugt. Auf diese Weise ist es möglich, ohne zusätzliche Komponenten allein durch die strömende Spülflüssigkeit einen Unterdruck zu erzeugen. Dies ist eine einfache, sehr robuste und zuverlässige Lösung.

Grundsätzlich wäre es auch denkbar, eine Verengung auch an einem anderen Bereich der Medientrenneinrichtung, welcher von der Spülflüssigkeit durchströmt wird, vorzusehen, um den Unterdruck an jenem anderen Bereich entstehen zu lassen. Der Strömungspfad würde dann in diesen anderen Bereich münden, um für ein effektives Ansaugen eines nicht nur flüssigen Fluids aus der Aufbereitungskammer durch die Medientrenneirichtung zu sorgen.

In einer Variante weist die Medientrenneinrichtung eine erste Kammer auf, die in Strömungsverbindung mit dem ersten Einlass gebracht werden kann. Dabei wird die erste Kammer vorzugsweise durch zwei Ventilsitze begrenzt. Ein erster Ventilsitz befindet sich dabei an einem ersten Ende der ersten Kammer, und ein zweiter Ventilsitz an einem zweiten Ende der ersten Kammer. Das zweite Ende der ersten Kammer liegt dabei dem ersten Ende der ersten Kammer gegenüber. Zwischen den beiden Ventilsitzen ist in dieser Variante ein Ventilkörper angeordnet, der beweglich ausgestaltet ist und sich zwischen dem ersten Ventilsitz und dem zweiten Ventilsitz hin- und her bewegen kann. Der erste Ventilsitz kann beispielsweise am ersten Einlass für Spülflüssigkeit angeordnet sein. Wenn der Ventilkörper dann auf dem ersten Ventilsitz sitzt, ist der Einlass für Spülflüssigkeit verschlossen; es kann keine Spülflüssigkeit in die Medientrenneinrichtung eindringen. In diesem Betriebszustand der Medientrenneinrichtung kann dann hingegen Trocknungsluft durch den zweiten Einlass für Trocknungsluft in die Medientrenneinrichtung eintreten.

Wenn der Ventilkörper auf dem zweiten Ventilsitz sitzt, verschließt er hingegen einen zur Zuführung von Trocknungsluft in die erste Kammer vorgesehenen Durchlass. Dabei wird der Ventilkörper insbesondere durch den Druck von der durch den ersten Einlass in die Medientrenneinrichtung eintretenden Spülflüssigkeit gegen den zweiten Ventilsitz gedrückt. Umgekehrt wird der Ventilkörper insbesondere durch den Luftdruck der durch den zweiten Einlass in die Medientrenneinrichtung eintretenden Trocknungsluft gegen den ersten Ventilsitz gedrückt, wenn keine Spülflüssigkeit in die Medientrenneinrichtung eintritt.

Der Ventilkörper ist dabei insbesondere gleitend ausgestaltet, sodass er innerhalb der ersten Kammer zwischen dem ersten Ventilsitz und dem zweiten Ventilsitz hin- und hergleiten kann. Zu diesem Zweck kann der Ventilkörper mindestens eine Gleitfläche aufweisen. Insbesondere ist es vorgesehen, dass eine Unterseite und eine Oberseite des Ventilkörpers als Gleitflächen ausgestattet sind, während eine rechte Seitenfläche und eine linke Seitenfläche zum Inkontaktbringen mit dem ersten Ventilsitz und dem zweiten Ventilsitz ausgestaltet sind.

Wenn der Ventilkörper von der Spülflüssigkeit gegen den zweiten Ventilsitz gedrückt wird, wird dadurch effektiv ein Eindringen von Spülflüssigkeit in den zweiten Einlass der Medientrenneinrichtung vermieden. Wenn der Ventilkörper durch die Trocknungsluft gegen den ersten Ventilsitz gedrückt wird, wird effektiv ein Eindringen von Trocknungsluft in den Einlass für Spülflüssigkeit vermieden. Durch das Hin- und Hergleiten des Ventilkörpers wird also erreicht, dass die Spülflüssigkeit vom ersten Einlass zum gemeinsamen Auslass strömen kann, während die Trocknungsluft aus dem zweiten Einlass zum gemeinsamen Auslass strömen kann.

In einer Variante ist zumindest ein Ventilsitz, beispielsweise alle Ventilsitze, vollständig aus Kunststoff hergestellt. Dabei können unterschiedliche Grundstoffe für eine äußere Komponente und eine innere Komponente des entsprechenden Ventilsitzes verwendet werden, die in einem gemeinsamen Herstellungsverfahren verarbeitet werden.

In einer Variante weist einer der Ventilsitze eine integrierte Halterung für die dritte Ventileinrichtung auf. Ferner weist dieser Ventilsitz vorzugsweise eine integrierte Halterung für einen Magneten auf, mit dem die dritte Ventileinrichtung an dem Ventilsitz gehalten werden kann, um die zur Öffnung der dritten Ventileinrichtung erforderliche Kraft zu erhöhen.

In einer Variante ist es angedacht, dass die Ventilsitze durch Rastbleche fixiert werden.

In einer Variante weist der Ventilkörper einen Hohlraum auf, der einen Abschnitt des Strömungspfades bildet, der dazu dient, dass ein von der Vorrichtung zur Erzeugung eines Unterdrucks angesaugtes nicht nur flüssiges Fluid durch ihn hindurchströmen kann.

In einer Variante weist die Medientrenneinrichtung eine zweite Kammer auf, die mit dem zweiten Einlass in Strömungsverbindung gebracht werden kann. Es ist vorzugsweise vorgesehen, dass der Strömungspfad die zweite Kammer mit einem von der Spülflüssigkeit durchströmten Bereich der Medientrenneinrichtung verbindet. Damit ist es möglich, dass durch das aktive Ansaugen eines entsprechenden, desinfizierend wirkenden Fluids eine Desinfektion der zweiten Kammer und des Strömungspfads, der die zweite Kammer mit dem von der Spülflüssigkeit durchströmten Bereich der Medientrenneinrichtung verbindet, erreicht wird. Sofern sich Leckageflüssigkeit in der zweiten Kammer ansammelt, wird diese also durch das aktive Ansaugen des Fluids desinfiziert, so dass beim anschließenden Durchströmen von Trocknungsluft durch die zweite Kammer keine Substanzen mitgerissen werden, die die medizinischen und/oder zahnmedizinischen Instrumente, die in der Aufbereitungskammer des Reinigungs- und Desinfektionsgeräts aufbereitet wurden, rekontaminieren könnten.

Dabei lässt sich die Medientrenneinrichtung in äußerst kompakter Bauweise herstellen, sodass sie innerhalb des Reinigungs- und Desinfektionsgeräts nur einen geringen Platzbedarf hat.

Wenn sich der Strömungspfad vorzugsweise auch durch den Hohlraum des Ventilkörpers erstreckt, wird folglich nicht nur die zweite Kammer von dem angesaugten Fluid durchströmt, sondern auch der Hohlraum innerhalb des Ventilkörpers. Zu diesem Zweck weist der Ventilkörper insbesondere einen der zweiten Kammer zugewandten Fluideinlass und einen der zweiten Kammer abgewandten Fluidauslass auf. Der Fluideinlass kann dabei über eine Ventileinrichtung, beispielsweise über eine Rückschlagklappe, verschlossen werden, die es gestattet, dass ein Fluid aus der zweiten Kammer durch den Ventilkörper strömt, nicht jedoch in umgekehrter Richtung in die zweite Kammer strömen kann. Der Fluidauslass kann beispielsweise mit einem weiteren Abschnitt des Strömungspfades in Strömungsverbindung gebracht werden, der selbst in einen Bereich mündet, der von Spülflüssigkeit durchströmt werden kann, wie beispielsweise in den gemeinsamen Auslass.

In einer Variante ist der Ventilkörper ein- oder mehrteilig aus Kunststoff ausgeführt. In einer anderen Variante weist der Ventilkörper zumindest einen Kunststoff auf. Beispielsweise kann der Ventilkörper aus einem Metall und einem Kunststoff bestehen.

In einer Variante weist der Ventilkörper eine integrierte Halterung für ein Dichtelement und/oder für die erste Ventileinrichtung auf.

In einer weiteren Variante ist in der zweiten Kammer eine zweite Ventileinrichtung angeordnet, die im geschlossenen Zustand einen Durchbruch zwischen der zweiten Kammer und der Aufbereitungskammer überdeckt. Durch diesen Durchbruch kann bei geöffneter zweiter Ventileinrichtung ein Fluid aus der Aufbereitungskammer in die zweite Kammer gesaugt werden, wenn ein entsprechender Unterdruck an der zweiten Kammer angelegt wird. Die zweite Ventileinrichtung ist dabei derart ausgelegt, dass sie den Durchbruch auch im geschlossenen Zustand nicht vollständig verschließt. Vielmehr gestattet sie auch in ihrem geschlossenen Zustand noch ein Abfließen einer Flüssigkeit aus der zweiten Kammer in die Aufbereitungskammer. Wenn sich also in der zweiten Kammer beispielsweise eine aus der Aufbereitungskammer angesaugte Flüssigkeit ansammelt, kann diese durch den Durchbruch stets wieder in die Aufbereitungskammer zurückfließen. Daher ist der Durchbruch vorzugsweise in einem Boden der zweiten Kammer angeordnet, sodass ein Abfließen von Flüssigkeit aus der zweiten Kammer durch den Durchbruch mittels Schwerkraft gewährleistet werden kann.

In einer Variante ist in der zweiten Kammer eine dritte Ventileinrichtung angeordnet, die die zweite Kammer im geschlossenen Zustand von dem zweiten Einlass fluiddicht trennt. Die dritte Ventileinrichtung ist als Rückschlageinrichtung ausgestaltet und kann durch ein Fluid, welches sich in der zweiten Kammer befindet, nicht geöffnet werden. Das heißt, ein Fluidübertritt aus der zweiten Kammer zum zweiten Einlass der Medientrenneinrichtung wird verhindert. Demgegenüber ist es für ein aus dem zweiten Einlass zur zweiten Kammer hin strömendes Fluid möglich, die dritte Ventileinrichtung zu öffnen.

In einer Variante ist in der Medientrenneinrichtung eine Vorrichtung vorgesehen, die mit der dritten Ventileinrichtung in Wirkverbindung steht und eine Kraft auf die dritte Ventileinrichtung ausübt, um das Öffnen der dritten Ventileinrichtung zu erschweren. Bei dieser Vorrichtung zur Ausübung einer Kraft kann es sich beispielsweise um eine Feder handeln, die die dritte Ventileinrichtung auf die geschlossene Position vorspannt. Die Vorrichtung kann auch eine magnetische Vorrichtung sein, die die dritte Ventileinrichtung in ihrem geschlossenen Zustand hält. Wenn dann Trocknungsluft durch den zweiten Einlass in die Medientrenneinrichtung strömt, muss die von dieser Vorrichtung zur Ausübung einer Kraft auf die dritte Ventileinrichtung ausgeübte Kraft überwunden werden, um die dritte Ventileinrichtung zu öffnen. Auf diese Weise wird ein unbeabsichtigtes Öffnen der dritten Ventileinrichtung vermieden. Vielmehr wird die dritte Ventileinrichtung erst dann geöffnet, wenn Trocknungsluft mit einem ausreichend hohen Druck durch den zweiten Einlass in die Medientrenneinrichtung eingeführt wird.

Die erste Ventileinrichtung und die dritte Ventileinrichtung sind vorzugsweise in Serie angeordnet, sodass ein unerwünschtes Rückströmen von Spülflüssigkeit oder einem anderen Fluid aus der Medientrenneinrichtung zum zweiten Einlass hin vermieden wird.

In einer Variante ist ein Ventilsitz, der zur Begrenzung der ersten Kammer und/oder der zweiten Kammer dient, in Form eines Zwei-Komponenten-Elements ausgebildet. Dabei ist eine innere Komponente, die insbesondere unelastisch ausgeführt sein kann, vorgesehen, die von einer äußeren, elastischen Komponente zumindest abschnittsweise umgeben ist. Die äußere Komponente kann die innere Komponente beispielsweise vollständig umgeben. Das Zwei-Komponenten-Element kann beispielsweise als Spritzgussteil hergestellt werden. Die elastische Komponente kann ein Elastomer aufweisen oder aus einem Elastomer bestehen. Es ist denkbar und vorgesehen, die äußere elastische Komponente in Übermaß herzustellen, sodass die äußere Komponente bei einem Einbau in ein Gehäuse der Medientrenneinrichtung eine Kompression erfährt und auf diese Weise eine dichtende Wirkung entfaltet. Dabei ist in dem Ventilsitz eine Fluiddurchführung ausgebildet, durch die ein Fluid (beispielsweise Spülflüssigkeit oder Trocknungsluft) strömen kann.

In einer Variante ist es vorgesehen, dass die Medientrenneinrichtung drei Ventilsitze aufweist. Dabei begrenzt ein Ventilsitz nur die erste Kammer und ein Ventilsitz nur die zweite Kammer, während ein dritter Ventilsitz sowohl die erste Kammer als die zweite Kammer begrenzt, da er zwischen beiden Kammern angeordnet ist.

Die Ventilsitze können insbesondere in steckbarer Form ausgebildet sein, sodass sie in das Gehäuse der Medientrenneinrichtung einfach eingesteckt werden können.

Ferner ist zumindest einer der Ventilsitze vorzugsweise derart ausgestaltet, dass er in den von der Spülflüssigkeit durchströmten Bereichen der Medientrenneinrichtung ein restloses Ablaufen der Spülflüssigkeit ermöglicht. Dies kann über eine glatte Ausgestaltung derjenigen Flächen des Ventilsitzes, die mit Spülflüssigkeit in Kontakt kommen, bewerkstelligt werden. Ferner kann es sinnvoll sein, den Ventilsitz an den Stellen, an denen er mit Spülflüssigkeit in Kontakt kommt, mit einer dauerhaften hydrophoben Beschichtung auszustatten oder ihn aus einem hydrophoben Material herzustellen, um ein einfaches Ablaufen der Spülflüssigkeit von dem Ventilsitz zu ermöglichen.

In einer Variante ist die äußere elastische Komponente des Ventilsitzes an ihrer Außenseite zumindest abschnittsweise mit umlaufenden Lamellen versehen. Die umlaufenden Lamellen können in einer Variante auch den gesamten Ventilsitz umgeben. Durch eine derartige lamellenartige Ausgestaltung der äußeren, elastischen Komponente des Ventilsitzes ist es besonders einfach möglich, diesen in Übermaß zu fertigen und bei einer Montage eine dichtende Kompression auf den Ventilsitz auszuüben.

In einer Variante sind die Lamellen konisch ausgeführt, das heißt, eine äußere Kontur der mit Lamellen versehenen Bereiche der äußeren elastischen Komponente weist eine konische Ausgestaltung auf. Durch diese konische Ausgestaltung können Toleranzen besser ausgeglichen werden, so dass eine sicherere Abdichtung und eine leichtere Montage des Ventilsitzes möglich sind. Außerdem zentriert sich der Ventilsitz bei dieser Ausgestaltung bei der Montage von selbst.

In einer Variante weist die Medientrenneinrichtung einen Grundkörper auf, der mit einer Innenseite einer Wandung der Aufbereitungskammer des Reinigungs- und Desinfektionsgeräts verbunden ist. Ein solcher Grundkörper kann auch als Grundplatte bezeichnet werden. Der Grundkörper bildet vorzugsweise den Boden der Medientrenneinrichtung. Dabei ist es insbesondere vorgesehen, dass sich eine Längserstreckungsrichtung der Medientrenneinrichtung horizontal erstreckt. Der Grundkörper kann gegenüber der Wandung der Aufbereitungskammer mittels eines geeigneten Dichtelements, beispielsweise einer kombinierten Flächen-/Rundschnurdichtung, abgedichtet sein, sodass kein unbeabsichtigter Fluidaustritt aus der Aufbereitungskammer erfolgen kann.

In einer Variante ist der Grundkörper einteilig ausgestaltet. In einer weiteren Variante weist der Grundkörper integrierte Befestigungselemente auf, mittels deren eine Befestigung an der Wandung der Aufbereitungskammer möglich ist. Dabei sind die Befestigungselemente vorzugsweise von der Aufbereitungskammer aus nicht sichtbar. Ferner sind die Befestigungselemente vorzugsweise nicht medienberührend ausgestaltet. Das heißt, sie kommen im Betrieb des Reinigungs- und Desinfektionsgeräts nicht mit Spülflüssigkeit, Trocknungsluft oder einem anderen Medium aus der Aufbereitungskammer in Kontakt.

In einer Variante bildet der Grundkörper zusammen mit dem ersten Ventilsitz und dem zweiten Ventilsitz eine Führung für den Ventilkörper, die den zur Bewegung des Ventilkörpers zur Verfügung stehenden Raum definiert.

In einer weiteren Variante ist die zweite Ventileinrichtung, die für ein Abdecken des Durchbruchs sorgt, welcher die zweite Kammer mit der Aufbereitungskammer verbindet, in den Grundkörper integriert. Dies erleichtert sowohl die Herstellung als auch die Montage der entsprechenden Elemente.

In einer weiteren Variante ist der gemeinsame Auslass im Grundkörper ausgebildet und als Drehführung für eine Fluidabgabevorrichtung ausgestaltet. Die Fluidabgabevorrichtung befindet sich dabei in der Aufbereitungskammer. Beispielsweise kann es sich bei der Fluidabgabevorrichtung um einen drehbaren Spülarm handeln. Wenn die entsprechende Drehführung bereits in dem Grundkörper ausgebildet ist, entfällt die zusätzliche Herstellung einer separaten Drehführung, was abermals die Montage des gesamten Reinigungs- und Desinfektionsgeräts erleichtert.

In einer Variante steht der gemeinsame Auslass in Strömungsverbindung mit mindestens einer ersten Fluidabgabevorrichtung und mindestens einer zweiten Fluidabgabevorrichtung. Die erste Fluidabgabevorrichtung dient dabei einer Behandlung einer Außenseite von in der Aufbereitungskammer angeordneten medizinischen und/oder zahnmedizinischen Instrumenten. Beispielsweise kann es sich bei der ersten Fluidabgabevorrichtung um einen Spülarm handeln, der die Außenseite der zu behandelnden Instrumente mit Spülflüssigkeit oder Trocknungsluft beaufschlagt. Die zweite Fluidabgabevorrichtung dient einer Behandlung der Innenseite von in der Aufbereitungskammer angeordneten medizinischen und/oder zahnmedizinischen Instrumenten. Beispielsweise kann die zweite Fluidabgabevorrichtung als Spülkorb mit Adaptern zum Anschluss von medizinischen und/oder zahnmedizinischen Instrumenten ausgestaltet sein, über die die Innenlumina oder anderen Innenbereiche der entsprechenden Instrumente mit Spülflüssigkeit oder Trocknungsluft beaufschlagt werden können. In dieser Variante ist es besonders einfach möglich, sowohl die Außenseiten als auch die Innenseiten der zu behandelnden Instrumente erst mit Spülflüssigkeit und anschließend mit Trocknungsluft zu beaufschlagen, um so für eine effiziente Reinigung, Desinfektion und Trocknung der Instrumente zu sorgen.

Mit der erfindungsgemäß beanspruchten Lösung wird ein bei einer aktiven Trocknung aufbereiteter Instrumente bestehendes hygienisches Risiko signifikant reduziert, da eine von Trocknungsluft durchströmte Kammer während des gewöhnlichen Betriebsablaufs des Reinigungs- und Desinfektionsgeräts stets mit desinfiziert wird. Ferner wird der geräteseitige Trocknungsstrang, in dem beispielsweise ein Gebläse und eine Heizung angeordnet sind, effektiv vor einem Eintritt von Leckageflüssigkeit geschützt. Darüber hinaus wird die Trocknungsleistung des Reinigung- und Desinfektionsgeräts in Bezug auf Hohlinstrumente verbessert, da eine Erhöhung des Luftstromes innerhalb der Lumina der Instrumente erreicht wird. Denn mit der Medientrenneinrichtung des vorliegend beanspruchten Reinigungs- und Desinfektionsgeräts wird die gesamte Trocknungsluft durch einen gemeinsamen Auslass aus der Medientrenneinrichtung zu entsprechenden Instrumenten befördert. Ein Druckabfall durch ein Ausströmen von Trocknungsluft durch eine andere Öffnung der Medientrenneinrichtung wird effektiv vermieden.

Schließlich wird die Trocknungsleistung des Reinigungs- und Desinfektionsgeräts in Bezug auf englumige und insbesondere in der Aufbereitungskammer stehend angeordnete Instrumente verbessert, da es durch die verbesserte Zufuhr von Trocknungsluft möglich ist, eine in einem Hohlraum eines Instruments stehende Wassersäule gegen den hydrostatischen Druck auszutreiben.

Die der Erfindung zugrunde liegende Aufgabe wird auch durch ein Verfahren zum Desinfizieren eines von einem Fluid durchströmbaren Bereichs eines Reinigungs- und Desinfektionsgeräts für medizinische und/oder zahnmedizinische Instrumente gelöst. Dabei ist der von dem Fluid durchströmbare Bereich außerhalb einer Aufbereitungskammer des Reinigungs- und Desinfektionsgeräts angeordnet.

Das erfindungsgemäß beanspruchte Verfahren zeichnet sich dadurch aus, dass beim Strömen von Spülflüssigkeit ein Unterdruck erzeugt wird. Dieser Unterdruck wird dazu eingesetzt, ein nicht nur flüssiges Fluid, welches desinfizierende Eigenschaften aufweist, durch den Bereich zu saugen. In einer Variante wird das nicht nur flüssige Fluid aus der Aufbereitungskammer des Reinigungs- und Desinfektionsgeräts gesaugt.

Bei den aus dem Stand der Technik bekannten Verfahren ist ein derartiges aktives Saugen eines desinfizierenden Fluids, welches sich ohnehin in der Aufbereitungskammer befindet, nicht bekannt. Vielmehr können sich bei den aus dem Stand der Technik bekannten Lösungen stets Leckageflüssigkeitsansammlungen beispielsweise in einem Trocknungsluftzufuhrbereich ergeben. Dort ist dann eine Keimbildung möglich, die nachfolgend zu einer Rekontamination des bereits aufbereiteten Spülguts führt, wenn Trocknungsluft durch eine entsprechende Medientrenneinrichtung zum aufbereiteten Spülgut geführt wird.

Der zu desinfizierende Bereich kann beispielsweise ein beliebiger Abschnitt eines Umwälzkreislaufs des Reinigungs- und Desinfektionsgeräts sein. Dabei ist insbesondere eine Desinfektion von Toträumen innerhalb des Umwälzkreislaufs von Interesse.

In einer Variante handelt es sich bei dem zu desinfizierenden Bereich um einen Trocknungsluftzufuhrbereich einer Medientrenneinrichtung eines Reinigungs- und Desinfektionsgeräts für medizinische und/oder zahnmedizinische Instrumente. Dabei weist die Medientrenneinrichtung einen ersten Einlass für Spülflüssigkeit auf, der in einer Variante mit einer ersten Kammer in Strömungsverbindung gebracht werden kann. Ferner weist die Medientrenneinrichtung einen zweiten Einlass für Trocknungsluft auf, der in einer Variante mit einer zweiten Kammer in Strömungsverbindung gebracht werden kann. Schließlich ist ein gemeinsamer Auslass für Spülflüssigkeit und Trocknungsluft in der Medientrenneinrichtung ausgebildet, durch den hindurch entweder Spülflüssigkeit oder Trocknungsluft aus der Medientrenneinrichtung strömen kann.

Das Verfahren kann beispielsweise derart ausgeführt werden, dass beim Strömen von Spülflüssigkeit durch die Medientrenneinrichtung ein Unterdruck in der Medientrenneinrichtung erzeugt wird, mittels dessen das nicht nur flüssige Fluid mit desinfizierenden Eigenschaften aus der Aufbereitungskammer in die Medientrenneinrichtung gesaugt wird und dabei den Trocknungsluftzufuhrbereich der Medientrenneinrichtung durchströmt.

In einer Variante wird das nicht nur flüssige Fluid mit desinfizierenden Eigenschaften durch die zweite Kammer und durch einen Strömungspfad, der die zweite Kammer mit der strömenden Spülflüssigkeit verbindet, gesaugt. Auf diese Weise wird eine effiziente Desinfektion der zweiten Kammer, welche der Zufuhr von Trocknungsluft durch die Medientrenneinrichtung in die Aufbereitungskammer des Reinigungs- und Desinfektionsgerät dient, erreicht.

Die im Zusammenhang mit dem beschriebenen Reinigungs- und Desinfektionsgerät erläuterten Varianten und beispielhaften Ausgestaltungen sind in beliebiger Weise miteinander kombinierbar und in beliebiger Weise auf das beschriebene Verfahren zum Desinfizieren eines Trocknungsluftzufuhrbereichs übertragbar, und umgekehrt.

Aspekte der vorliegenden Erfindung werden nachfolgend unter Bezugnahme auf Ausführungsbeispiele und entsprechende Figuren näher erläutert. Es zeigen:
- Figur 1: eine schematische Schnittansicht durch ein Ausführungsbeispiel eines Reinigungs- und Desinfektionsgeräts mit einer Medientrenneinrichtung;
- Figur 2: eine Schnittansicht durch ein Ausführungsbeispiel einer Medientrenneinrichtung in einem ersten Betriebszustand;
- Figur 3: eine Schnittansicht durch die Medientrenneinrichtung der Figur 2 in einem zweiten Betriebszustand;
- Figur 4A: eine Darstellung eines Ausführungsbeispiels einer Schottwand und
- Figur 4B: eine Querschnittsdarstellung der Schottwand der Figur 4A.

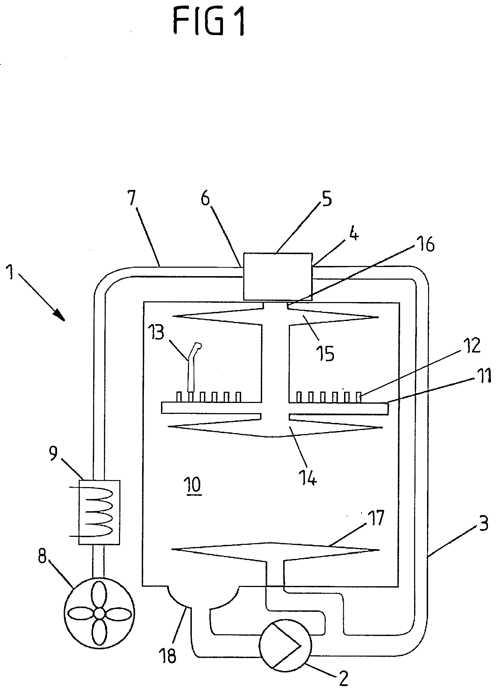

Die Figur 1 zeigt ein Reinigungs- und Desinfektionsgerät 1 in einer schematischen Querschnittsansicht. Eine Umwälzpumpe 2 beschickt einen Spülflüssigkeitsstrang 3 mit Spülflüssigkeit. Diese gelangt an einem Spülflüssigkeitszugang 4, der auch als erster Einlass bezeichnet werden kann, in einen Medientrenner 5. An den Medientrenner 5 ist zudem über einen Luftzugang 6, der auch als zweiter Einlass bezeichnet werden kann, ein Trocknungsstrang 7 angeschlossen. Der Trocknungsstrang 7 dient dazu, Trocknungsluft, die von einem Gebläse 8 in den Trocknungsstrang 7 befördert und von einer Trocknungsheizung 9 innerhalb des Trocknungsstrangs 7 erwärmt wird, dem Medientrenner 5 zuzuführen.

Der Medientrenner 5 dient dazu, die unterschiedlichen Medien - also Spülflüssigkeit auf der einen Seite und Trocknungsluft auf der anderen Seite - voneinander zu trennen und nacheinander in eine Aufbereitungskammer 10 des Reinigungs- und Desinfektionsgeräts 1 einzubringen. Dazu ist innerhalb der Aufbereitungskammer 10 ein Beladungskorb 11 angeordnet, der mit Adaptern 12 zum Aufsetzen von medizinischen und/oder zahnmedizinischen Instrumenten 13 ausgestattet ist. Dabei sind die Adapter 12 derart ausgestaltet, dass Spülflüssigkeit oder Trocknungsluft, die durch den Beladungskorb 11 zu den Adaptern 12 geführt wird, einen Innenbereich der medizinischen und/oder zahnmedizinischen Instrumente 13 durchströmen kann. Darüber hinaus sind ein mittlerer Spülarm 14 und ein oberer Spülarm 15 ebenfalls mit dem Medientrenner 5 verbunden und können von Spülflüssigkeit oder Trocknungsluft durchströmt werden. Auf diese Weise ist es möglich, die medizinischen und/oder zahnmedizinischen Instrumente 13 nicht nur von ihrer Innenseite her zu reinigen, zu desinfizieren und zu trocknen, sondern auch von ihrer Außenseite. Der strömungstechnische Anschluss des Beladungskorbs 11, des mittleren Spülarms 14 und des oberen Spülarms 15 erfolgt dabei an einem gemeinsamen Medienauslasss 16 des Medientrenners 5.

In der Aufbereitungskammer 10 ist zudem ein unterer Spülarm 17 angeordnet, der nicht mit dem Medientrenner 5 verbunden ist, sondern unmittelbar an den Spülflüssigkeitsstrang 3 angeschlossen ist. Das heißt, dass keine Trocknungsluft durch den unteren Spülarm 17 in das Innere der Aufbereitungskammer 10 geleitet werden kann.

Wenn die Instrumente 13 in der Aufbereitungskammer 10 von Spülflüssigkeit durchspült werden, sammelt sich die Spülflüssigkeit anschließend in einem Pumpensumpf 18, der mit der Umwälzpumpe 2 verbunden ist. Die Umwälzpumpe 2 kann anschließend die Spülflüssigkeit wieder aus dem Pumpensumpf 18 ansaugen und erneut in den Spülflüssigkeitsstrang 3 einbringen.

Wenn Spülflüssigkeit über den Spülflüssigkeitsstrang 3 in den Medientrenner 5 eingebracht wird, wird im Medientrenner 5 ein Ventil geschlossen, das den Medientrenner 5 mit dem Trocknungsstrang 7 verbindet. Umgekehrt wird im Medientrenner 5 ein anderes Ventil geschlossen, wenn Trocknungsluft durch den Trocknungsstrang 7 in den Medientrenner 5 einströmt, sodass aus dem gemeinsamen Auslass 16 des Medientrenners 5 entweder Spülflüssigkeit oder Trocknungsluft strömen kann. Dabei sorgt ein Verschließen des Trocknungsstrangs 7 beim Einströmen von Spülflüssigkeit über den Spülflüssigkeitsstrang 3 in den Medientrenner 5 dafür, dass kein unerwünschter Eintrag von Spülflüssigkeit in den Trocknungsstrang 7 erfolgt. Umgekehrt sorgt ein Verschließen des Spülflüssigkeitsstrangs 3 beim Einströmen von Trocknungsluft durch den Trocknungsstrang 7 in den Medientrenner 5 dafür, dass die Trocknungsluft nur durch den gemeinsamen Medienauslass 16 durch die Adapter 12 und die Innenbereiche der Instrumente 13 sowie durch den mittleren Spülarm 14 und den oberen Spülarm 15 strömen kann. Trocknungsluft, die durch die Innenbereiche der Instrumente 13 strömt, tritt anschließend ebenfalls in die Aufbereitungskammer 10 ein und sorgt - wie die Luft, die aus dem mittleren Spülarm 14 und dem oberen Spülarm 15 austritt - für ein Trocknen der Außenseite der Instrumente 13.

Darüber hinaus sorgt die Trocknungsluft, die durch den mittleren Spülarm 14 und den oberen Spülarm 15 strömt, für ein Trocknen der Innenbereiche dieser Spülarme.

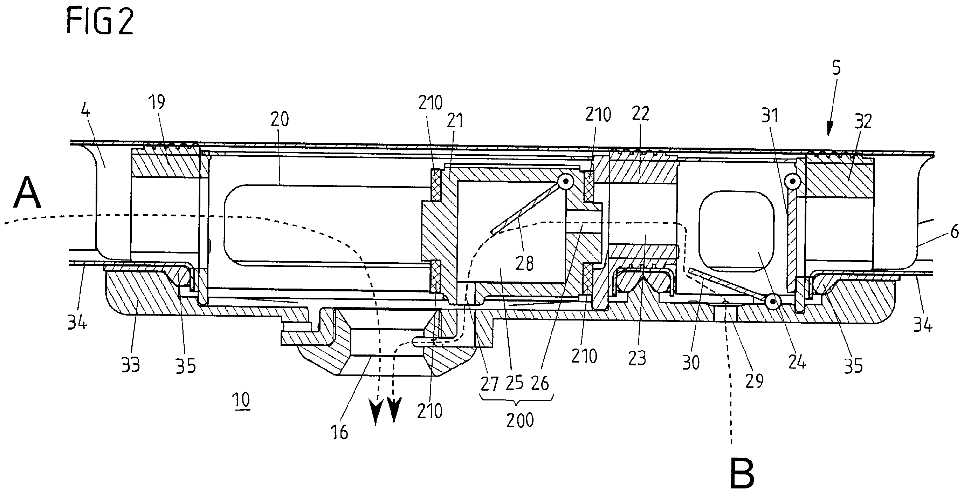

Die Figur 2 zeigt eine Querschnittsansicht durch den Medientrenner 5 der Figur 1. Dabei werden gleiche Elemente mit den gleichen Bezugszeichen wie in der Figur 1 versehen.

Auf der linken Seite des Medientrenners 5 ist der Spülflüssigkeitszugang 4 dargestellt, der in der Abbildung der Figur 1 rechts dargestellt ist. Auf der rechten Seite des Medientrenners 5 befindet sich in der Darstellung der Figur 2 der Luftzugang 6. Wenn Spülflüssigkeit durch den Spülflüssigkeitszugang 4 in den Medientrenner 5 einströmt, durchströmt sie zuerst eine erste Schottwand 19, die als erster Ventilsitz dient. Anschließend gelangt die Spülflüssigkeit in eine erste Kammer 20, aus der die Spülflüssigkeit durch den gemeinsamen Medienauslass 16 wieder aus dem Medientrenner 5 austreten kann. Der Weg, den die Spülflüssigkeit durch den Medientrenner 5 nimmt, ist mit einer mit A gekennzeichneten gestrichelten Linie schematisch dargestellt.

Innerhalb der ersten Kammer 20 ist ein Ventilkörper 21 angeordnet. Dieser Ventilkörper 21 sitzt in der Darstellung der Figur 2 auf einer zweiten Schottwand 22, die als zweiter Ventilsitz fungiert. Damit verschließt der Ventilkörper 21 einen Durchbruch 23, der eine Strömungsverbindung 23 zwischen der ersten Kammer 20 und einer zweiten Kammer 24 ermöglicht. Zur besseren Abdichtung gegenüber der ersten Schottwand 19 und der zweiten Schottwand 22 weist der Ventilkörper 21 auf seinen Stirnseiten zwei Dichtungen 210 auf.

Der Ventilkörper 21 weist einen Hohlraum 25 auf, der über einen Fluideinlass 26 mit dem Durchbruch 23 verbunden ist und damit mit der zweiten Kammer 24 in Strömungsverbindung steht. Der Ventilkörper 21 weist ferner einen Fluidauslass 27 auf, der in Strömungsverbindung mit dem gemeinsamen Medienauslasss 16 steht. Der Fluideinlass 26, der Hohlraum 25 und der Fluidauslass 27 bilden einen Strömungspfad 200, mit dem die zweite Kammer 24 mit dem gemeinsamen Medienauslasss 16 strömungstechnisch verbunden ist.

Innerhalb des Ventilkörpers 21 ist zudem eine erste Rückschlagklappe 28 angeordnet, die als erste Ventileinrichtung dient und den Fluideinlass 26 des Ventilkörpers 21 verschließen kann. Diese erste Rückschlagklappe 28 sorgt dafür, dass ein Rückströmen eines Fluids durch den Fluidausgang 27 und den Hohlraum 25 zum Fluideinlass 26 und damit zur zweiten Kammer 24 verhindert wird.

Am Boden der zweiten Kammer 24 ist zudem eine Öffnung 29 vorgesehen, die eine Strömungsverbindung zwischen der zweiten Kammer 24 und der Aufbereitungskammer 10 des Reinigungs- und Desinfektionsgeräts 1 bildet. Diese Öffnung 29 kann von einer zweiten Rückschlagklappe 30, die als zweite Ventileinrichtung dient, überdeckt werden. Dabei lässt die zweite Rückschlagklappe 30 jedoch in ihrem vollständig geschlossenen Zustand einen kleinen Spalt geöffnet, sodass sich Flüssigkeit, die sich innerhalb der zweiten Kammer 24 ansammelt, durch die Öffnung 29 wieder zurück in die Aufbereitungskammer 10 strömen kann.

Wenn nun Spülflüssigkeit entlang des mit A gekennzeichneten Spülflüssigkeitspfads strömt, wird in dem gemeinsamen Auslass 16 ein Unterdruck erzeugt. Denn dieser gemeinsame Auslass 16 ist gegenüber dem vorherigen Strömungsquerschnitt verengt; er bildet eine Venturi-Düse. Dadurch wird ein Unterdruck erzeugt, der sich über den Fluidauslass 27, den Hohlraum 25 und den Fluideinlass 26 bis zum Durchbruch 23 und zur zweiten Kammer 24 auswirkt. Durch diesen Unterdruck kann Dampf oder feucht-heiße Luft aus der Aufbereitungskammer 10 durch die Öffnung 29 in die zweite Kammer 24 und von dort durch den Strömungspfad 200 weiter zum gemeinsamen Medienauslass 16 gesaugt werden. Dies ist schematisch mit einer mit B gekennzeichneten gestrichelten Linie dargestellt. Durch das Einströmen von Dampf bzw. feucht-heißer Luft aus der Aufbereitungskammer 10 kommt es zur effizienten Desinfektion der zweiten Kammer 24 und der weiteren von dem Dampf bzw. der feucht-heißen Luft durchströmten Bereiche des Medientrainers 5.

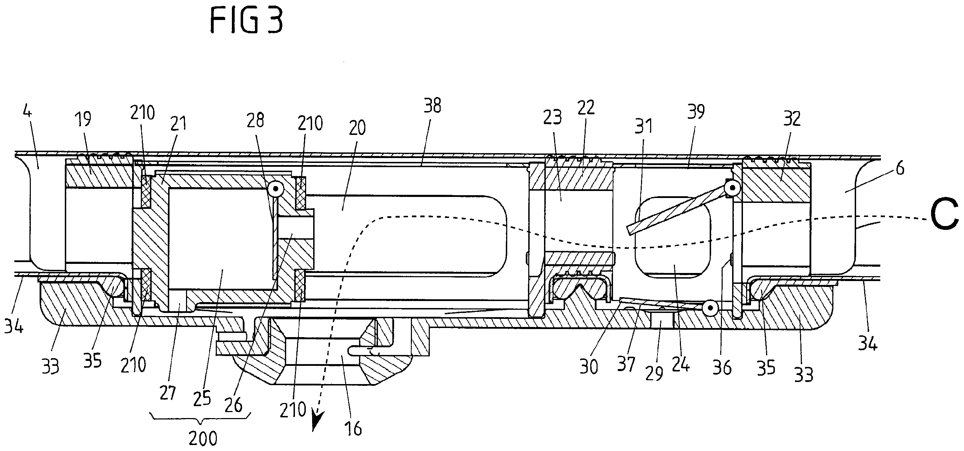

Während des in der Figur 2 gezeigten Betriebszustands des Medientrenners 5, in dem Spülflüssigkeit durch den Medientrenner 5 strömt, ist eine dritte Rückschlagklappe 31, die an einer dritten Schottwand 32 befestigt ist, geschlossen. Dabei wird ein Schließen der dritten Rückschlagklappe 31 einerseits durch das Eigengewicht der dritten Rückschlagklappe 31 und der damit auf sie wirkenden Schwerkraft sowie andererseits über eine Magnetkraft erreicht, die von einem an der dritten Schottwand 32 befestigten Magneten (vgl. Figur 3) auf die dritte Rückschlagklappe 31 ausgeübt wird. Ein Einströmen von Trocknungsluft durch den Luftzugang 6 ist in diesem Betriebszustand nicht möglich.

Der Medientrenner 5 weist eine Grundplatte 33 auf, die als Grundkörper dient. Diese Grundplatte 33 ist gegen eine Innenwand 34 der Aufbereitungskammer 10 gedrückt. Dabei wird über eine kombinierte Flächen-/Rundschnurdichtung 35 eine Abdichtung zwischen der Grundplatte 33 und der inneren Wandung 34 der Aufbereitungskammer 10 erreicht.

Die Figur 3 zeigt den Medientrenner 5 aus der Figur 2 in einem zweiten Betriebszustand. Es werden dieselben Bezugszeichen für dieselben Elemente wie in der Figur 2 verwendet; darüber hinaus wird auf die Erläuterungen zur Figur 2 verwiesen.

In dem in der Figur 3 dargestellten Betriebszustand des Medientrenners 5 strömt Trocknungsluft durch den Medientrenner 5. Nach Abschalten der Umwälzpumpe und Ausbleiben eines Spülflüssigkeitsdrucks wird der Ventilkörper 21 nicht mehr gegen die zweite Schottwand 22 gedrückt. Vielmehr ist es nun möglich, den Ventilkörper 21 durch Trocknungsluft, die durch den Luftzugang 6 in den Medientrenner 5 einströmt, gegen die erste Schottwand 19 zu drücken, die als erster Ventilsitz dient. Dadurch wird die erste Kammer 20 gegenüber dem Spülflüssigkeitszugang 4 abgedichtet.

Die Trocknungsluft weist einen hinreichend großen Druck auf, um die dritte Rückschlagklappe 31 zu öffnen. Im geöffneten Zustand der Rückschlagklappe 31 ist ein Magnet 36 an der dritten Schottwand 32 zu sehen, auf den bereits bei der Beschreibung der Figur 2 Bezug genommen wurde. Dieser Magnet 36 dient zur Ausübung einer erhöhten Rückhaltekraft auf die dritte Rückschlagklappe 31. Die in den Medientrenner 5 einströmende Trocknungsluft strömt durch die zweite Kammer 24, den Durchbruch 23 und die erste Kammer 20 hin zum gemeinsamen Medienauslass 16. Dabei wird am gemeinsamen Medienauslass 16 zwar wieder ein Unterdruck erzeugt. Aufgrund des verschobenen Ventilkörpers 21 mündet dieser Unterdruck jedoch in der ersten Kammer 20 und sorgt damit nicht mehr für ein Einströmen eines Fluids aus der Aufbereitungskammer 10 durch die Öffnung 29 im Boden der zweiten Kammer 24. Vielmehr ist diese Öffnung 29 durch die zweite Rückschlagklappe 30 nun verschlossen, wobei, wie bereits erwähnt, ein kleiner Spalt 37 verbleibt, durch den Flüssigkeit, welche sich in der zweiten Kammer 24 angesammelt hat, durch die Öffnung 29 zurück in die Aufbereitungskammer 10 strömen kann.

Da die zweite Kammer 24 im vorherigen Betriebszustand des Medientrenners 5 von heißem Dampf durchströmt wurde, ist sie desinfiziert. Wenn nun die Trocknungsluft durch die zweite Kammer 24 strömt, kann sie keine Bestandteile mitreißen, die die in der Aufbereitungskammer 10 angeordneten Instrumente rekontaminieren könnten.

Da die Trocknungsluft ausschließlich über den gemeinsamen Auslass 16 in die Aufbereitungskammer 10 strömt (der in der zweiten Kammer 24 verbleibende Schlitz 37 zum Ablauf von Flüssigkeit durch die Öffnung 29 ist derart dimensioniert, dass aufgrund der Druckverhältnisse keine Trocknungsluft durch die Öffnung 29 in die Aufbereitungskammer 10 strömen kann, denn die durchströmende Luft drückt auf die zweite Rückschlagkappe 30, so dass diese angepresst wird und die Öffnung 29 abdichtet), wird die Trocknungsluft 10 mit hohem Druck gezielt auf die Innenseiten und Außenseiten der in der Aufbereitungskammer 10 angeordneten Instrumente geleitet.

Die erste Schottwand 19 und die zweite Schottwand 22 stellen die seitlichen Begrenzungen der ersten Kammer 20 dar. Darüber hinaus stellen die zweite Schottwand 22 und die dritte Schottwand 32 die seitlichen Begrenzungen der zweiten Kammer 24 dar. Mithin ist die zweite Schottwand 22 zwischen der ersten Kammer 20 und der zweiten Kammer 24 angeordnet und bildet jeweils eine Seite jeder Kammer. Die erste Schottwand 19, die zweite Schottwand 22 und die dritte Schottwand 32 sind in den Medientrenner 5 eingesteckt und werden dabei durch ein erstes Rastblech 38 und zweites Rastblech 39 fixiert.

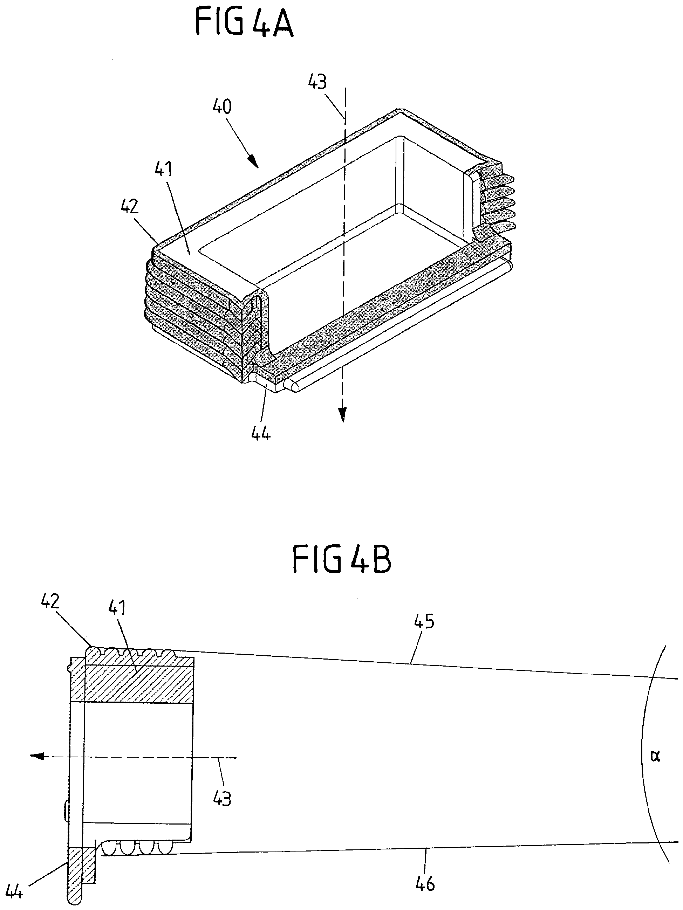

Eine mögliche Ausgestaltung der ersten Schottwand 19, der zweiten Schottwand 22 und/oder der dritten Schottwand 32 ist in den Figuren 4A und 4B im Detail dargestellt. So zeigt die Figur 4A eine Ansicht auf eine Schottwand 40, die als Zwei-Komponenten-Spritzgussteil hergestellt wurde. Eine innere Komponente 41 besteht dabei aus einem unelastischen Kunststoff, während eine äußere Komponente 42 aus einem elastischen Kunststoff besteht. Die äußere Komponente 42 ist dabei in Lamellenform um die innere Komponente 41 herum angeordnet.

Die Schottwand 40 weist einen Hohlraum auf, durch den entlang des mit 43 bezeichneten und gestrichelt dargestellten Pfeils ein Medium wie Spülflüssigkeit oder Trocknungsluft strömen kann.

In einem seitlich abstehenden Bereich 44 der Schottwand 40 ist ein Anschlag für einen Ventilkörper ausgebildet, sodass die Schottwand 40 als Ventilsitz geeignet ist.

Die Figur 4B zeigt die Schottwand 40 der Figur 4A in einer Querschnittsdarstellung, wobei dieselben Elemente mit denselben Bezugszeichen wie in der Figur 4A versehen sind. Es wird darüber hinaus auf die Erläuterungen zur Figur 4A verwiesen. In der Darstellung der Figur 4B ist zu sehen, dass die in Lamellenform ausgestaltete äußere Komponente eine konisch geformte Außenkontur aufweist. Legt man virtuelle Linien 45, 46 beidseits an die quergeschnittene Außenkontur der äußeren Komponente 42 an, verlaufen diese virtuellen Linien 45, 46 nicht parallel, sondern in einem Winkel α zueinander.

## Patentansprüche

1. Reinigungs- und Desinfektionsgerät für medizinische und/oder zahnmedizinische Instrumente, mit einer Medientrenneinrichtung (5) und einer Aufbereitungskammer (10), wobei die Medientrenneinrichtung (5) einen ersten Einlass (4) für Spülflüssigkeit, einen zweiten Einlass (6) für Trocknungsluft sowie einen gemeinsamen Auslass (16) für Spülflüssigkeit und Trocknungsluft aufweist, durch den hindurch wahlweise Spülflüssigkeit oder Trocknungsluft aus der Medientrenneinrichtung (5) strömen kann,
**dadurch gekennzeichnet,**
**dass** die Medientrenneinrichtung (5) eine Vorrichtung zur Erzeugung eines Unterdrucks aufweist, mittels der ein nicht nur flüssiges Fluid in die Medientrenneinrichtung (5) gesaugt werden kann.

2. Reinigungs- und Desinfektionsgerät nach Anspruch 1, **dadurch gekennzeichnet, dass** die Vorrichtung zur Erzeugung eines Unterdrucks im gemeinsamen Auslass (16) ausgebildet ist.

3. Reinigungs- und Desinfektionsgerät nach Anspruch 1 oder 2, **dadurch gekennzeichnet, dass** die Vorrichtung zur Erzeugung eines Unterdrucks als Venturi-Düse ausgestaltet ist.

4. Reinigungs- und Desinfektionsgerät nach einem der vorherigen Ansprüche, **dadurch gekennzeichnet, dass** es eine mit dem ersten Einlass (4) für Spülflüssigkeit in Strömungsverbindung bringbare erste Kammer (20) aufweist, die auf einer ersten Seite durch einen ersten Ventilsitz (19) und auf einer der ersten Seite gegenüberliegenden zweiten Seite durch einen zweiten Ventilsitz (22) begrenzt wird, wobei ein beweglich ausgestalteter Ventilkörper (21) zwischen dem ersten Ventilsitz (19) und dem zweiten Ventilsitz (22) angeordnet ist.

5. Reinigungs- und Desinfektionsgerät nach Anspruch 4, **dadurch gekennzeichnet, dass** der Ventilkörper (21) einen Hohlraum (25) aufweist, der einen Abschnitt eines Strömungspfades (200) bildet, der dazu dient, dass ein von der Vorrichtung zur Erzeugung eines Unterdrucks angesaugtes nicht nur flüssiges Fluid durch ihn hindurchströmen kann.

6. Reinigungs- und Desinfektionsgerät nach einem der vorherigen Ansprüche, **dadurch gekennzeichnet, dass** es eine mit dem zweiten Einlass (6) für Trocknungsluft in Strömungsverbindung bringbare zweite Kammer (24) aufweist, wobei ein Strömungspfad (200), der dazu dient, dass ein von der Vorrichtung zur Erzeugung eines Unterdrucks angesaugtes nicht nur flüssiges Fluid durch ihn hindurchströmen kann, die zweite Kammer (24) mit einem von Spülflüssigkeit durchströmbaren Bereich der Medientrenneinrichtung verbindet.

7. Reinigungs- und Desinfektionsgerät nach Anspruch 5 und 6, **dadurch gekennzeichnet, dass** innerhalb des Ventilkörpers (21) eine erste Ventileinrichtung (28) angeordnet ist, die ein Strömen eines Fluids durch den Ventilkörper (21) nur von der zweiten Kammer (24) weg ermöglicht.

8. Reinigungs- und Desinfektionsgerät nach Anspruch 6 oder 7, **dadurch gekennzeichnet, dass** in der zweiten Kammer (24) eine zweite Ventileinrichtung (30) angeordnet ist, die im geschlossenen Zustand einen Durchbruch (29) zwischen der zweiten Kammer (24) und der Aufbereitungskammer (10) überdeckt, dabei aber eine Strömungsverbindung (37) zwischen der zweiten Kammer (24) und der Aufbereitungskammer (10) gewährleistet, um den Übertritt einer Flüssigkeit aus der zweiten Kammer (24) in die Aufbereitungskammer (10) zu ermöglichen.

9. Reinigungs- und Desinfektionsgerät nach einem der vorherigen Ansprüche, **dadurch gekennzeichnet, dass** ein Ventilsitz (19, 22, 32), der der Begrenzung einer mit dem ersten Einlass (4) für Spülflüssigkeit in Strömungsverbindung bringbaren ersten Kammer (20) und/oder einer mit dem zweiten Einlass (6) für Trocknungsluft in Strömungsverbindung bringbaren zweite Kammer (24) dient, als Zwei-Komponenten-Element ausgebildet ist, wobei eine äußere elastische Komponente (42) eine innere Komponente (41) zumindest abschnittsweise umgibt.

10. Reinigungs- und Desinfektionsgerät nach Anspruch 9, **dadurch gekennzeichnet, dass** die äußere elastische Komponente (42) an ihrer Außenseite zumindest abschnittsweise umlaufende Lamellen aufweist.

11. Reinigungs- und Desinfektionsgerät nach einem der vorherigen Ansprüche, **dadurch gekennzeichnet, dass** die Medientrenneinrichtung (5) einen Grundkörper (33) aufweist, der an einer Innenseite der Aufbereitungskammer (10) mit einer Wandung (34) der Aufbereitungskammer (10) verbunden ist.

12. Reinigungs- und Desinfektionsgerät nach Anspruch 11, **dadurch gekennzeichnet, dass** der gemeinsame Auslass (16) im Grundkörper (33) ausgebildet und als Drehführung für eine in der Aufbereitungskammer (10) angeordnete drehbare Fluidabgabevorrichtung (14, 15) ausgestaltet ist.

13. Reinigungs- und Desinfektionsgerät nach einem der vorherigen Ansprüche, **dadurch gekennzeichnet, dass** der gemeinsame Auslass (16) in Strömungsverbindung mit mindestens einer ersten Fluidabgabevorrichtung (14, 15), die einer Behandlung einer Außenseite von in der Aufbereitungskammer angeordneten medizinischen und/oder zahnmedizinischen Instrumenten (13) dient, und mindestens einer zweiten Fluidabgabevorrichtung (12), die einer Behandlung einer Innenseite von in der Aufbereitungskammer angeordneten medizinischen und/oder zahnmedizinischen Instrumenten (13) dient, steht.

14. Verfahren zum Desinfizieren eines von einem Fluid durchströmbaren Bereichs eines Reinigungs- und Desinfektionsgeräts (1) für medizinische und/oder zahnmedizinische Instrumente, wobei der Bereich außerhalb einer Aufbereitungskammer (10) des Reinigungs- und Desinfektionsgeräts (1) angeordnet ist,
**dadurch gekennzeichnet,**
**dass** durch ein Strömen von Spülflüssigkeit ein Unterdruck erzeugt wird, mittels dessen ein nicht nur flüssiges Fluid mit desinfizierenden Eigenschaften durch den Bereich gesaugt wird.

15. Verfahren nach Anspruch 14, **dadurch gekennzeichnet, dass** der Bereich ein Trocknungsluftzufuhrbereich einer Medientrenneinrichtung (5) des Reinigungs- und Desinfektionsgeräts (1) ist, wobei die Medientrenneinrichtung (5) einen ersten Einlass (4) für Spülflüssigkeit, einen zweiten Einlass (6) für Trocknungsluft sowie einen gemeinsamen Auslass (16) für Spülflüssigkeit und Trocknungsluft aufweist, durch den hindurch wahlweise Spülflüssigkeit oder Trocknungsluft aus der Medientrenneinrichtung strömen kann.

## Geänderte Patentansprüche

### Geänderte Patentansprüche gemäss Regel 137(2) EPÜ.

1. Reinigungs- und Desinfektionsgerät für medizinische und/oder zahnmedizinische Instrumente, mit einer Medientrenneinrichtung (5) und einer Aufbereitungskammer (10), wobei die Medientrenneinrichtung (5) einen ersten Einlass (4) für Spülflüssigkeit, einen zweiten Einlass (6) für Trocknungsluft sowie einen gemeinsamen Auslass (16) für Spülflüssigkeit und Trocknungsluft aufweist, durch den hindurch wahlweise Spülflüssigkeit oder Trocknungsluft aus der Medientrenneinrichtung (5) strömen kann,
**dadurch gekennzeichnet,**
**dass** die Medientrenneinrichtung (5) eine Vorrichtung zur Erzeugung eines Unterdrucks aufweist, mittels der ein nicht nur flüssiges Fluid mit desinfizierenden Eigenschaften aus der Aufbereitungskammer in einen Trocknungsluftzufuhrbereich der Medientrenneinrichtung (5) und durch die Medientrenneinrichtung (5) hindurch gesaugt werden kann.

2. Reinigungs- und Desinfektionsgerät nach Anspruch 1, **dadurch gekennzeichnet, dass** die Vorrichtung zur Erzeugung eines Unterdrucks im gemeinsamen Auslass (16) ausgebildet ist.

3. Reinigungs- und Desinfektionsgerät nach Anspruch 1 oder 2, **dadurch gekennzeichnet, dass** die Vorrichtung zur Erzeugung eines Unterdrucks als Venturi-Düse ausgestaltet ist.

4. Reinigungs- und Desinfektionsgerät nach einem der vorherigen Ansprüche, **dadurch gekennzeichnet, dass** es eine mit dem ersten Einlass (4) für Spülflüssigkeit in Strömungsverbindung bringbare erste Kammer (20) aufweist, die auf einer ersten Seite durch einen ersten Ventilsitz (19) und auf einer der ersten Seite gegenüberliegenden zweiten Seite durch einen zweiten Ventilsitz (22) begrenzt wird, wobei ein beweglich ausgestalteter Ventilkörper (21) zwischen dem ersten Ventilsitz (19) und dem zweiten Ventilsitz (22) angeordnet ist.

5. Reinigungs- und Desinfektionsgerät nach Anspruch 4, **dadurch gekennzeichnet, dass** der Ventilkörper (21) einen Hohlraum (25) aufweist, der einen Abschnitt eines Strömungspfades (200) bildet, der dazu dient, dass ein von der Vorrichtung zur Erzeugung eines Unterdrucks angesaugtes nicht nur flüssiges Fluid durch ihn hindurchströmen kann.

6. Reinigungs- und Desinfektionsgerät nach einem der vorherigen Ansprüche, **dadurch gekennzeichnet, dass** es eine mit dem zweiten Einlass (6) für Trocknungsluft in Strömungsverbindung bringbare zweite Kammer (24) aufweist, wobei ein Strömungspfad (200), der dazu dient, dass ein von der Vorrichtung zur Erzeugung eines Unterdrucks angesaugtes nicht nur flüssiges Fluid durch ihn hindurchströmen kann, die zweite Kammer (24) mit einem von Spülflüssigkeit durchströmbaren Bereich der Medientrenneinrichtung verbindet.

7. Reinigungs- und Desinfektionsgerät nach Anspruch 5 und 6, **dadurch gekennzeichnet, dass** innerhalb des Ventilkörpers (21) eine erste Ventileinrichtung (28) angeordnet ist, die ein Strömen eines Fluids durch den Ventilkörper (21) nur von der zweiten Kammer (24) weg ermöglicht.

8. Reinigungs- und Desinfektionsgerät nach Anspruch 6 oder 7, **dadurch gekennzeichnet, dass** in der zweiten Kammer (24) eine zweite Ventileinrichtung (30) angeordnet ist, die im geschlossenen Zustand einen Durchbruch (29) zwischen der zweiten Kammer (24) und der Aufbereitungskammer (10) überdeckt, dabei aber eine Strömungsverbindung (37) zwischen der zweiten Kammer (24) und der Aufbereitungskammer (10) gewährleistet, um den Übertritt einer Flüssigkeit aus der zweiten Kammer (24) in die Aufbereitungskammer (10) zu ermöglichen.

9. Reinigungs- und Desinfektionsgerät nach einem der vorherigen Ansprüche, **dadurch gekennzeichnet, dass** ein Ventilsitz (19, 22, 32), der der Begrenzung einer mit dem ersten Einlass (4) für Spülflüssigkeit in Strömungsverbindung bringbaren ersten Kammer (20) und/oder einer mit dem zweiten Einlass (6) für Trocknungsluft in Strömungsverbindung bringbaren zweite Kammer (24) dient, als Zwei-Komponenten-Element ausgebildet ist, wobei eine äußere elastische Komponente (42) eine innere Komponente (41) zumindest abschnittsweise umgibt.

10. Reinigungs- und Desinfektionsgerät nach Anspruch 9, **dadurch gekennzeichnet, dass** die äußere elastische Komponente (42) an ihrer Außenseite zumindest abschnittsweise umlaufende Lamellen aufweist.

11. Reinigungs- und Desinfektionsgerät nach einem der vorherigen Ansprüche, **dadurch gekennzeichnet, dass** die Medientrenneinrichtung (5) einen Grundkörper (33) aufweist, der an einer Innenseite der Aufbereitungskammer (10) mit einer Wandung (34) der Aufbereitungskammer (10) verbunden ist.

12. Reinigungs- und Desinfektionsgerät nach Anspruch 11, **dadurch gekennzeichnet, dass** der gemeinsame Auslass (16) im Grundkörper (33) ausgebildet und als Drehführung für eine in der Aufbereitungskammer (10) angeordnete drehbare Fluidabgabevorrichtung (14, 15) ausgestaltet ist.

13. Reinigungs- und Desinfektionsgerät nach einem der vorherigen Ansprüche, **dadurch gekennzeichnet, dass** der gemeinsame Auslass (16) in Strömungsverbindung mit mindestens einer ersten Fluidabgabevorrichtung (14, 15), die einer Behandlung einer Außenseite von in der Aufbereitungskammer angeordneten medizinischen und/oder zahnmedizinischen Instrumenten (13) dient, und mindestens einer zweiten Fluidabgabevorrichtung (12), die einer Behandlung einer Innenseite von in der Aufbereitungskammer angeordneten medizinischen und/oder zahnmedizinischen Instrumenten (13) dient, steht.

14. Verfahren zum Desinfizieren eines von einem Fluid durchströmbaren Bereichs eines Reinigungs- und Desinfektionsgeräts (1) für medizinische und/oder zahnmedizinische Instrumente, wobei der Bereich außerhalb einer Aufbereitungskammer (10) des Reinigungs- und Desinfektionsgeräts (1) angeordnet ist,
**dadurch gekennzeichnet,**
**dass** der Bereich ein Trocknungsluftzufuhrbereich einer Medientrenneinrichtung (5) des Reinigungs- und Desinfektionsgeräts (1) ist, wobei die Medientrenneinrichtung (5) einen ersten Einlass (4) für Spülflüssigkeit, einen zweiten Einlass (6) für Trocknungsluft sowie einen gemeinsamen Auslass (16) für Spülflüssigkeit und Trocknungsluft aufweist, durch den hindurch wahlweise Spülflüssigkeit oder Trocknungsluft aus der Medientrenneinrichtung strömen kann, und dass durch ein Strömen von Spülflüssigkeit ein Unterdruck erzeugt wird, mittels dessen ein nicht nur flüssiges Fluid mit desinfizierenden Eigenschaften aus der Aufbereitungskammer durch den Trocknungsluftzufuhrbereich gesaugt wird.
